# EUROPEAN PATENT SPECIFICATION

(11) **EP 3 914 293 B1**
(45) Date of publication and mention of the grant of the patent: **03.07.2024**
(21) Application number: 21705092.1
(22) Date of filing: 20.01.2021
(51) Int. Cl.: A61K 39/12, A61P 31/14

(54) **VACCINATION AGAINST CORONAVIRUS WITH POLIOMYELITIS VACCINE**
IMPFUNG GEGEN CORONAVIRUS MIT POLIOMYELITIS-IMPFSTOFF
VACCINATION CONTRE LE CORONAVIRUS AVEC UN VACCIN CONTRE LA POLIOMYÉLITE

(30) Priority: 11.04.2020 US 202063008664 P; 15.04.2020 US 202063010678 P; 22.04.2020 US 202063013561 P; 15.11.2020 US 202017098449
(43) Date of publication of application: 01.12.2021
(73) Proprietor: Xie, Qiyi, San Diego, CA 92121 (US)
(72) Inventor: Xie, Qiyi, San Diego, CA 92121 (US)
(74) Representative: Maiwald GmbH
(86) International application number: PCT/US2021/014253
(87) International publication number: WO 2021/206783

(56) References cited:
- US-A1- 2003 190 331
- US-A1- 2008 193 478
- US-A1- 2008 193 478
- US-A1- 2017 321 192
- US-A1- 2020 000 825
- US-A1- 2020 108 136
- Anonymous: "Australia's Trialing a TB Vaccine Against COVID-19, And Health Workers Get It First", Science Alert, 27 March 2020 (2020-03-27), XP055730631, Retrieved from the Internet: URL:https://www.sciencealert.com/australia -is-trialling-a-tb-vaccine-for-coronavirus -and-health-workers-get-it-first [retrieved on 2020-09-15]
- DE BREE L C J ET AL: "Non-specific effects of vaccines: Current evidence and potential implications", SEMINARS IN IMMUNOLOGY, W.B. SAUNDERS COMPANY, PA, US, vol. 39, 11 July 2018 (2018-07-11), pages 35-43, XP085548746, ISSN: 1044-5323, DOI: 10.1016/J.SMIM.2018.06.002
- CHUMAKOV KONSTANTIN ET AL: "Can existing live vaccines prevent COVID-19?", SCIENCE, vol. 368, no. 6496, 12 June 2020 (2020-06-12), pages 1187-1188, XP055886930, US ISSN: 0036-8075, DOI: 10.1126/science.abc4262
- Amanpour And Company: "EXCLUSIVE: Can an Oral Polio Vaccine Help Stop the Coronavirus?", , 13 April 2020 (2020-04-13), XP055887211, Retrieved from the Internet: URL:https://www.youtube.com/watch?v=azTcdQ KcKvw

## Description

### CROSS REFERENCE TO RELATED APPLICATIONS

This application claims the benefit of the priority of U.S. Provisional Application Nos. 63/008,664, filed April 11, 2020; 63/010,678, filed April 15, 2020; and 63/013,561, filed April 22, 2020; and U.S. Application No. 17/098,449, filed November 15, 2020; the disclosure of each of which is incorporated herein by reference in its entirety.

### FIELD

Provided herein is a poliomyelitis vaccine for use in preventing a person from an infection by a *Coronaviridae* virus. Also provided herein is a poliomyelitis vaccine for use in inducing a protective immune response in a person against a *Coronaviridae* virus.

### REFERENCE TO A SEQUENCE LISTING

The present specification is being filed with a Sequence Listing in Computer Readable Form (CRF), which is entitled 805A002WO01_SEQ_LISTING_ST25.txt of 20,153 bytes in size and created January 20, 2021; the content of which is incorporated herein by reference in its entirety.

### BACKGROUND

Coronavirus disease 2019 (COVID-19) is caused by severe acute respiratory syndrome coronavirus-2 (SARS-CoV-2). Gorbalenya et al., Nat. Microbiol. 2020, 5, 536-44; Zhang et al., Science 2020, 368, 409-12. On March 11, 2020, the World Health Organization declared COVID-19 a global pandemic. Zhang et al., Science 2020, 368, 409-12; Dai et al., Science 2020, 368, 1331-5. By mid-July of 2020, COVID-19 has spread almost to every corner of the world. As of October 2020, there are almost 50 million confirmed cases and over a million confirmed deaths globally. The COVID-19 pandemic poses as a great public health threat to the world.

Oral poliovirus vaccine (OPV) could provide temporary protection against COVID-19 (Chumakov Konstantin et al., Science 2020, 368, 1187-1188).

COVID-19 can be fatal, especially to older people and those with pre-existing medical conditions. Shahid et al., J. Am. Geriatr. Soc. 2020, 68, 926-9. Common symptoms of COVID-19 include fever or chills, cough, shortness of breath or difficulty breathing, fatigue, muscle or body aches, headache, loss of taste or smell, sore throat, congestion or runny nose, nausea or vomiting, and diarrhea. *Id.* Currently, there is no FDA-approved vaccine for COVID-19. Therefore, there is an urgent need for an immunization method to battle the COVID-19 pandemic.

### SUMMARY OF THE DISCLOSURE

Provided herein is a poliomyelitis vaccine for use in (i) preventing a person from an infection by a Coronaviridae virus, (ii) inducing a protective immune response in a person against a Coronaviridae virus, (iii) inducing an immune response in a person protective against severe acute respiratory syndrome caused by an infection of a Coronaviridae virus, or (iv) reducing the severity of one or more symptoms of an infectious disease caused by a Coronaviridae virus in a person; wherein the poliomyelitis vaccine is an inactivated poliomyelitis vaccine.

### BRIEF DESCRIPTION OF THE DRAWINGS

FIG. 1 shows anti-SARS-CoV-2 RDRP antibody levels before and after poliovirus vaccine immunization of adult human subjects.
FIG. 2 shows anti-SARS-CoV-2 RDRP antibody levels before and during the COVID-19 pandemic from pediatric human subjects.

### DETAILED DESCRIPTION

The references to the methods of treatment by therapy in this description are to be interpreted as references to compounds, pharmaceutical compositions and medicaments of the present invention for use in those methods.

To facilitate understanding of the disclosure set forth herein, a number of terms are defined below.

Generally, the nomenclature used herein and the laboratory procedures in biochemistry, biology, immunology, virology, and pharmacology described herein are those well-known and commonly employed in the art. Unless defined otherwise, all technical and scientific terms used herein generally have the same meaning as commonly understood by one of ordinary skill in the art to which this disclosure belongs.

The terms "treat," "treating," and "treatment" are meant to include alleviating or abrogating a disorder, disease, or condition, or one or more of the symptoms associated with the disorder, disease, or condition; or alleviating or eradicating the cause(s) of the disorder, disease, or condition itself.

The terms "prevent," "preventing," and "prevention" are meant to include a method of delaying and/or precluding the onset of a disorder, disease, or condition, and/or its attendant symptoms; barring a person from acquiring a disorder, disease, or condition; or reducing a person's risk of acquiring a disorder, disease, or condition.

The terms "alleviate" and "alleviating" refer to easing or reducing one or more symptoms (*e*.*g*., pain) of a disorder, disease, or condition. The terms can also refer to reducing adverse effects associated with an active ingredient. Sometimes, the beneficial effects that a person derives from a prophylactic or therapeutic agent do not result in a cure of the disorder, disease, or condition.

The term "effective amount" or "immunologically effective amount" is meant to include the amount of a vaccine that, when administered, is sufficient to prevent development of, or alleviate to some extent, one or more of the symptoms of the disorder, disease, or condition being prevented or alleviated.

The term "about" or "approximately" means an acceptable error for a particular value as determined by one of ordinary skill in the art, which depends in part on how the value is measured or determined. In certain embodiments, the term "about" or "approximately" means within 1, 2, or 3 standard deviations. In certain embodiments, the term "about" or "approximately" means within 25%, 20%, 15%, 10%, 9%, 8%, 7%, 6%, 5%, 4%, 3%, 2%, 1%, 0.5%, or 0.05% of a given value or range.

Provided herein is a poliomyelitis vaccine for use in (i) preventing a person from an infection by a Coronaviridae virus, (ii) inducing a protective immune response in a person against a Coronaviridae virus, (iii) inducing an immune response in a person protective against severe acute respiratory syndrome caused by an infection of a Coronaviridae virus, or (iv) reducing the severity of one or more symptoms of an infectious disease caused by a Coronaviridae virus in a person; wherein the poliomyelitis vaccine is an inactivated poliomyelitis vaccine.

In certain embodiments, the *Coronaviridae* virus is a *Coronavirinae* virus. In certain embodiments, the *Coronaviridae* virus is a *Betacoronavirus.* In certain embodiments, the *Coronaviridae* virus is a SARS-CoV-1, SARS-CoV-2, or MERS-CoV. In certain embodiments, the *Coronaviridae* virus is a SARS-CoV-1. In certain embodiments, the *Coronaviridae* virus is a SARS-CoV-2. In certain embodiments, the *Coronaviridae* virus is a MERS-CoV.

In certain embodiments, the poliomyelitis vaccine is an inactivated poliomyelitis vaccine (IPV). In certain embodiments, the poliomyelitis vaccine is an inactivated poliomyelitis vaccine comprising inactivated Type 1 (Mahoney) poliovirus, inactivated Type 2 (MEF-1) poliovirus, inactivated Type 3 (Saukett) poliovirus, or a mixture thereof. In certain embodiments, the poliomyelitis vaccine comprises inactivated Type 1 poliovirus. In certain embodiments, the poliomyelitis vaccine comprises inactivated Type 2 poliovirus. In certain embodiments, the poliomyelitis vaccine comprises inactivated Type 3 poliovirus. In certain embodiments, the poliomyelitis vaccine comprises inactivated Type 1 poliovirus, inactivated Type 2 poliovirus, and inactivated Type 3 poliovirus.

In certain embodiments, the poliomyelitis vaccine is IPOL@. In certain embodiments, the poliomyelitis vaccine is an inactivated poliomyelitis vaccine comprising about 40 D antigen units of inactivated Type 1 poliovirus, about 8 D antigen units of inactivated Type 2 poliovirus, 32 D antigen units of inactivated Type 3 poliovirus, or a mixture thereof. In certain embodiments, the poliomyelitis vaccine is an inactivated poliomyelitis vaccine comprising about 40 D antigen units of inactivated Type 1 poliovirus. In certain embodiments, the poliomyelitis vaccine is an inactivated poliomyelitis vaccine comprising about 8 D antigen units of inactivated Type 2 poliovirus. In certain embodiments, the poliomyelitis vaccine is an inactivated poliomyelitis vaccine comprising 32 D antigen units of inactivated Type 3 poliovirus. In certain embodiments, the poliomyelitis vaccine is an inactivated poliomyelitis vaccine comprising about 40 D antigen units of inactivated Type 1 poliovirus, about 8 D antigen units of inactivated Type 2 poliovirus, and 32 D antigen units of inactivated Type 3 poliovirus.

In certain embodiments, the inactivated poliomyelitis vaccine is administered orally or parenterally. In certain embodiments, the inactivated poliomyelitis vaccine is administered orally. In certain embodiments, the inactivated poliomyelitis vaccine is administered parenterally. In certain embodiments, the inactivated poliomyelitis vaccine is administered intradermally. In certain embodiments, the inactivated poliomyelitis vaccine is administered intramuscularly. In certain embodiments, the inactivated poliomyelitis vaccine is administered subcutaneously.

In certain embodiments, the poliomyelitis vaccine is administered from about 1 to about 50 times, from about 1 to about 20 times, from about 1 to about 10, or from about 1 to about 5 times in a lifetime. In certain embodiments, the poliomyelitis vaccine is administered from about 1 to about 50 times in a lifetime. In certain embodiments, the poliomyelitis vaccine is administered from about 1 to about 20 times in a lifetime. In certain embodiments, the poliomyelitis vaccine is administered from about 1 to about 10 in a lifetime. In certain embodiments, the poliomyelitis vaccine is administered from about 1 to about 5 times in a lifetime.

In certain embodiments, the poliomyelitis vaccine is administered once or twice. In certain embodiments, the poliomyelitis vaccine is administered once. In certain embodiments, the poliomyelitis vaccine is administered once. In certain embodiments, the poliomyelitis vaccine is administered once as a booster dose. In certain embodiments, the poliomyelitis vaccine is administered once as a booster dose during a pandemic. In certain embodiments, the poliomyelitis vaccine is administered once as a booster dose during a COVID-19 pandemic.

In certain embodiments, the poliomyelitis vaccine is administered twice within about a year, about 6 months, about 3 months, about 2 months, about 1 months, or about 14 days. In certain embodiments, the poliomyelitis vaccine is administered twice within about a year. In certain embodiments, the poliomyelitis vaccine is administered twice within about 6 months. In certain embodiments, the poliomyelitis vaccine is administered twice within about 3 months. In certain embodiments, the poliomyelitis vaccine is administered twice within about 2 months. In certain embodiments, the poliomyelitis vaccine is administered twice within about 1 months. In certain embodiments, the poliomyelitis vaccine is administered twice within about 14 days.

In certain embodiments, the poliomyelitis vaccine is administered twice with an interval ranging from about 7 days to about 6 months, from about 7 days to about 3 months, from about 7 days to about 2 months, or from about 7 days to about 1 month. In certain embodiments, the poliomyelitis vaccine is administered twice with an interval ranging from about 7 days to about 6 months. In certain embodiments, the poliomyelitis vaccine is administered twice with an interval ranging from about 7 days to about 3 months. In certain embodiments, the poliomyelitis vaccine is administered twice with an interval ranging from about 7 days to about 2 months. In certain embodiments, the poliomyelitis vaccine is administered twice with an interval ranging from about 7 days to about 1 month.

In certain embodiments, the poliomyelitis vaccine is administered twice with the first dose as a priming dose and the second dose as a booster dose. In certain embodiments, the poliomyelitis vaccine is administered twice with both doses as booster doses.

In certain embodiments, the person to be given the poliomyelitis vaccine is unvaccinated against a poliovirus. In certain embodiments, the unvaccinated person receives 1, 2, or 3 doses of the poliomyelitis vaccine. In certain embodiments, the unvaccinated person receives 1 dose of the poliomyelitis vaccine. In certain embodiments, the unvaccinated person receives 2 doses of the poliomyelitis vaccine within about 1 or about two months. In certain embodiments, the unvaccinated person receives 3 doses of the poliomyelitis vaccine within about 3 months or about two years.

In certain embodiments, the person to be given the poliomyelitis vaccine is incompletely vaccinated against a poliovirus. In certain embodiments, the incompletely vaccinated person receives 1 or 2 doses of the poliomyelitis vaccine. In certain embodiments, the incompletely vaccinated person receives 1 dose of the poliomyelitis vaccine. In certain embodiments, the incompletely vaccinated person receives 2 doses of the poliomyelitis vaccine within about 1 or about two months.

In certain embodiments, the person to be given the poliomyelitis vaccine is completely vaccinated. In certain embodiments, the completely vaccinated person receives 1 dose of the poliomyelitis vaccine.

In certain embodiments, the immunologically effective amount of the inactivated poliomyelitis vaccine is ranging from about 1 to about 500, from about 2 to about 200, from about 5 to about 100, or from about 10 to about 100 D antigen units of an inactivated poliovirus. In certain embodiments, the immunologically effective amount of the inactivated poliomyelitis vaccine is ranging from about 1 to about 500 D antigen units of an inactivated poliovirus. In certain embodiments, the immunologically effective amount of the inactivated poliomyelitis vaccine is ranging from about 2 to about 200 D antigen units of an inactivated poliovirus. In certain embodiments, the immunologically effective amount of the inactivated poliomyelitis vaccine is ranging from about 5 to about 100 D antigen units of an inactivated poliovirus. In certain embodiments, the immunologically effective amount of the inactivated poliomyelitis vaccine is ranging from about 10 to about 100 D antigen units of an inactivated poliovirus.

The disclosure will be further understood by the following non-limiting examples.

### EXAMPLES

### Example 1

### Vaccination Against COVID-19 with Inactivated Poliomyelitis Vaccine

Four subjects each voluntarily took a booster dose of IPOL@ as recommended in the IPOL@ Label during the COVID-19 pandemic. Subject 1 (44-year-old, female) is a health care professional for more than 20 years. During the COVID-19 pandemic, Subject 1 had been exposed to COVID-19 on many occasions. Subject 1 and her family were planning to travel out of the country. Subject 1 encouraged the members of her family (Subject 2 - her husband (47-year-old), Subject 3 - her sister in her 50s, Subject 4 - her father (79-year-old), and Subject 5 - her mother (76-year-old)) to update all their immunizations prior to the travel. Subjects 1 and 3 to 5 received a booster dose of IPOL@ in June 2020 during the COVID19 pandemic, but Subject 2 decided not to receive a booster dose.

Subject 2 without receiving a booster dose became sick subsequently and was tested positive for COVID-19 shortly after. Subject 2 had since had unrelenting low-grade fevers (for more than five weeks), some confusion, and chronic weakness. Even though Subjects 1 and 3 to 5 had had frequent contact with Subject 2, none of them became ill.

Subjects 4 and 5, the parents of Subject 1, are elderly and in poor health. Subject 3, a sister of Subject 1, is also in poor health having a history of past stroke and cerebral palsy. All three of them were vaccinated and continued to do well with no signs of illness despite contact with Subject 2.

### Example 2

### Vaccination Against COVID-19 with Inactivated Poliomyelitis Vaccine

Subject A, a 70-year-old generally healthy female, received a booster dose of IPOL@ before travelling to Morocco in October 2019. Her husband, Subject B, a 69-year-old generally healthy male, received a booster dose of IPOL@ per advice of his physician on October 12, 2020. Subjects A and B were married and living together.

On October 14, 2020, their housekeeper had the symptoms of a dry throat, but continued to work in their house. While working in the house, the housekeeper wore a level 3 facemask, a face shield, and gloves. Subject A was in the house during the time when the housekeeper was working. The housekeeper was subsequently tested positive for COVID-19. The housekeeper's husband was also tested and found to be negative at the time.

Five days after her exposure to the housekeeper, Subject A awoke with a sore throat, which started on October 16, 2020 and ran through October 19, 2020. On October 19, 2020, she was tested positive for COVID-19. On October 20, 2020, her symptoms resolved and she was tested again but negative. Subject B, who received a poliomyelitis vaccine booster dose recently, was tested negative for COVID-19 on October 14 and 24, 2020.

In summary, the two elderly subjects (Subjects A and B) were exposed to a COVID-19-positive housekeeper. Subject A, who had a poliomyelitis vaccine booster dose a year ago, developed mild symptoms and tested positive for COVID-19 by a PCT test one day after exposure, but tested negative the following day using the same PCT test. Subject B, who had a poliomyelitis vaccine booster dose recently, never developed symptoms and tested negative during the time.

### Example 3

### Vaccination Against COVID-19 with Inactivated Poliomyelitis Vaccine

A working place outbreak of COVID-19 with 6 employees occurred in a health care related facility. Five of the employees received a booster dose of IPOL@, and the sixth employee, a young male adult, did not. Subsequently, the young adult contracted COVID-19 and brought the virus to the working place during the asymptomatic period. Few days later, he started showing some COVID-19 symptoms. The remaining five employees were tested positive for COVID-19 by PCR, but none of them showed any COVID-19 symptoms.

### Example 4 (comparative example)

### Vaccination Against COVID-19 with Attenuated Live Poliomyelitis Vaccine

On the first week of December 2020, an adult was inoculated with 2 drops of an OPV solution. The adult met six people in a New Year's Eve party in Malaysia. Few days later, all the six people contracted COVID-19. However, the male adult remained negative for COVID-19.

### Example 5 (comparative example)

### Vaccination Against COVID-19 with Attenuated Live Poliomyelitis Vaccine

On the first week of December 2020, all adults in a family were inoculated with 2 drops of an OPV solution, except a pregnant woman. After a New Year family reunion party, the pregnant woman contracted COVID-19 with minor symptoms, and her inoculated husband living with her was tested negative for COVID-19 and had no COVID-19 symptoms.

### Example 6

### Preparation of A Recombinant RDRP

A SARS-CoV-2 RDRP gene encoding region was amplified by PCR from a SARS-CoV-2 cDNA reverse-transcripted from a SARS-CoV-2 viral RNA and cloned into AMERIDX^{®} insect cell expression vector pADX50. After sequencing confirmation, the purified vector was transiently transfected into insect cell line Schneider 2 (S2). After induction by an ADX inducer, the RDRP protein expressed was purified by an affinity Ni-NTA column and further purified by DETA SEPHAROSE chromatography.

### Example 7

### Western Blot for Analyzing Human Anti-SARS-CoV-2 RDRP Antibodies

A recombinant poliovirus RDRP protein (SEQ ID NO: 1) and two recombinant SARS-CoV-2 RDRP proteins (SEQ ID NO: 2 and SEQ ID NO: 3) were mixed with an SDS-PAGE loading buffer and separated on a protein gel by electrophoresis. After the proteins on the gel were transferred onto a nitrocellulose membrane by electrophoresis, the membrane was blocked with 5% BSA and then treated with a diluted human serum sample to be analyzed for 30 mins. The membrane was washed and incubated with goat anti-human IgG/IgA/IgM conjugated with an HRP enzyme for 30 min. The membrane was washed and treated with a PIERCE^{™} ECL Western blotting substrate to generate chemiluminescence signals, which were detected and analyzed. Four subjects were analyzed using this assay and the results are summarized in Table 1, where the positive control is an individual who recently received a booster dose of IPOL@.

**TABLE 1**

| Subject (age, sex) | Before Poliovirus Vaccination | | | After Poliovirus Vaccination | | |
|---|---|---|---|---|---|---|
| | Poliovirus | Poliovirus RDRP | SARS-CoV-2 RDRP | Poliovirus | Poliovirus RDRP | SARS-CoV-2 RDRP |
| 12 yrs., M | | 1 | - | 3 | 3 | 2.5 |
| 14 yrs., F | 3 | 1 | - | 3 | 3 | 2.5 |
| 43 yrs., F | - | - | - | 3 | 3 | 2.5 |
| 47 yrs., M | - | - | - | 2 | 3 | 1.5 |
| Positive Control | | | | 3 | 3 | 2.5 |

### Example 8

### ELISA Assay for Analyzing Human Anti-SARS-CoV-2 RDRP Antibodies

A recombinant poliovirus RDRP protein (SEQ ID NO: 1) and two recombinant SARS-CoV-2 RDRP proteins (SEQ ID NO: 2 and SEQ ID NO: 3) were coated separately onto the wells of an ELISA plate by adding one of the proteins (100 µL) in a coating buffer (100 mM sodium carbonate) to a well. The ELISA plate was incubated at room temperature overnight or 4 hours at 37 °C and then blocked with 5% BSA in a phosphate-buffered saline solution. A diluted human serum or plasma to be analyzed was added to wells and the plate was incubated at room temperature. After washed with an ELISA washing buffer, the plate was incubated with goat anti-human IgG/IgA/IgM conjugated with an HRP enzyme. A TMB substrate was added. The reaction was stopped by adding 2 M sulfuric acid. The plate was read with an ELISA plate reader. Four human subjects were analyzed for anti-SARS-CoV-2 RDRP antibodies and the results are summarized in Table 2.

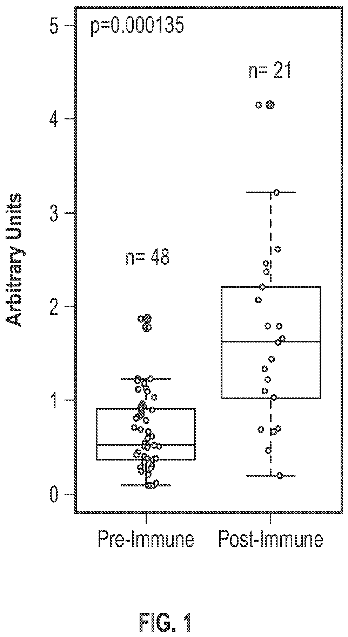

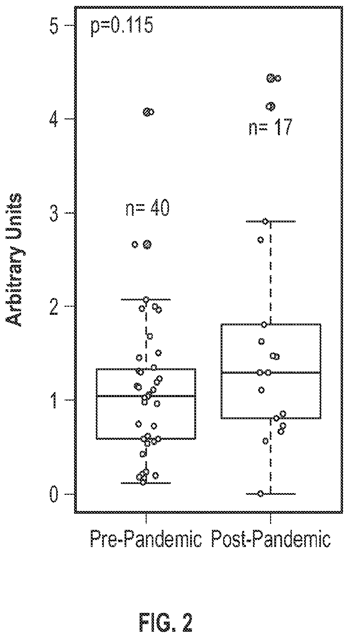

Additional sixty-nine serum samples from adult human subjects were analyzed for anti-SARS-CoV-2 RDRP antibodies, of which forty-eight were pre-poliovirus vaccination samples and twenty-one were post-poliovirus vaccination sample. For the analysis, each serum sample was diluted by 1:250. The results are shown in FIG. 1. For comparison, fifty-seven serum samples from pediatric subjects were also analyzed similarly for anti-SARS-CoV-2 RDRP antibodies, of which forty were pre-pandemic samples and seventeen were pandemic samples. The results are shown in FIG. 2.

**TABLE 2**

| Subject (age, sex) | Before Poliovirus Vaccination | After Poliovirus Vaccination |
|---|---|---|
| 12 yrs., M | 1 | 3 |
| 14 yrs., F | 1.5 | 2.5 |
| 43 yrs., F | 1 | 3 |
| 47 yrs., M | - | 2 |

### Example 9

### Antiviral Activity Against Live SARS-CoV-2

Four serum samples (Samples 1, 2, 3, and 4) were tested for their antiviral activity against a live SARS-CoV-2 (the MEX-BC2/2020 strain). Sample 1 was a serum sample from an individual under 5-year-old. Sample 2 was a serum sample from an individual under 5-year-old. Sample 3 was a serum from an individual (43 yrs., female) receiving a booster dose of IPOL^{®} in June 2020. Sample 4 was a serum from an individual (47 yrs., male) prior to the receipt of the booster dose. Two different assay protocols (Protocols 1 and 2) were used to evaluate antiviral activity.

### Antiviral Assay - Protocol 1:

To evaluate antiviral activity against SARS-CoV-2 (MEX-BC2/2020), a CPE-based antiviral assay was performed by infecting Vero E6 cells in the presence or absence of a serum sample. Infection of cells leads to significant cytopathic effect and cell death after 4 days of infection. In this assay, reduction of CPE in the presence of a serum sample was used to determine its antiviral activity. A viability assay was run in parallel for each serum sample.

Vero E6 cells were maintained in DMEM with 10% fetal bovine serum (FBS). The cells were seeded and incubated for 24 h before being pre-incubated with a serum sample. After cell culture was removed from the cells, each serum sample at serial dilutions was added to the cells and incubated for 1 h at 37 °C in a humidified incubator. The cells were then challenged with the viral inoculum resuspended in DMEM with 2% FBS. The amount of viral inoculum was previously titrated to result in a linear inhibitory response by an antiviral with activity against SARS-CoV-2. The cells were incubated in the presence of the virus inoculum and a serum sample for 96 h. The cell viability was then determined using the neutral red uptake assay.

The virus-induced CPE was monitored under the microscope after 3 days of infection and at day 4, the cells were stained with neutral red to determine cell viability. Viable cells incorporate neutral red in their lysosomes. The uptake relies on the ability of live cells to maintain the pH inside the lysosomes lower than in the cytoplasm. This process requires ATP. Inside the lysosome the dye becomes charged and is retained. After a 3 h incubation with neutral red (0.033%), the extra dye was washed and the neutral red taken by lysosomes was then extracted for 15 min with a solution containing 50% ethanol and 1% acetic acid to monitor absorbance at 540 nm.

Each serum sample was evaluated in duplicates using serial 2-fold dilutions. Controls included uninfected cells ("mock-infected"), and infected cells to which only vehicle was added. Full curves of positive plasma from convalescent patient (COV(+)) and GS-441524 were run as positive control inhibitors. GS-441524 is the main metabolite of remdesivir, a broad-spectrum antiviral that blocks the RNA polymerase of SARS-CoV-2.

The average absorbance at 540 nm (A540) observed in infected cells (in the presence of vehicle alone) was calculated and then subtracted from all samples to determine the inhibition of the virus induced CPE. Data points were then normalized to the average A540 signal observed in uninfected cells ("mock") after subtraction of the absorbance signal observed in infected cells. In the neutral red CPE-based assay, uninfected cells remained viable and uptake the dye at higher levels than non-viable cells. In the absence of an antiviral agent, the virus-induced CPE kills infected cells and leads to lower A540 (this value equals 0% inhibition). By contrast, incubation with an antiviral agent (COV(+) or GS-441524) prevents the virus induced CPE and leads absorbance levels similar to those observed in uninfected cells. Full recovery of cell viability in infected cells represent 100% inhibition of virus replication.

### Antiviral Assay - Protocol 2:

To evaluate antiviral activity against SARS-CoV-2 (MEX-BC2/2020), a CPE-based antiviral assay was performed by infecting Vero E6 cells in the presence or absence of a serum sample. Infection of cells leads to significant cytopathic effect and cell death after 4 days of infection. In this assay, reduction of CPE in the presence of a serum sample was used to determine its antiviral activity. A viability assay was run in parallel for each serum sample.

Vero E6 cells were maintained in DMEM with 10% fetal bovine serum (FBS). The cells were seeded and incubated for 24 h before being pre-incubated with virus. After cell culture was removed from the cells, the cells were challenged with the viral inoculum resuspended in DMEM with 2% FBS for 3 h at 37 °C in a humidified incubator. The amount of viral inoculum was previously titrated to result in a linear inhibitory response by an antiviral with activity against SARS-CoV-2. Each serum sample at serial dilutions was then added the infected cells. The cells were incubated in the presence of the virus inoculum and a serum sample for 96 h. The cell viability was then determined using the neutral red uptake assay.

The virus-induced CPE was monitored under the microscope after 3 days of infection and at day 4, the cells were stained with neutral red to determine cell viability. Viable cells incorporate neutral red in their lysosomes. The uptake relies on the ability of live cells to maintain the pH inside the lysosomes lower than in the cytoplasm. This process requires ATP. Inside the lysosome the dye becomes charged and is retained. After a 3 h incubation with neutral red (0.033%), the extra dye was washed and the neutral red taken by lysosomes was then extracted for 15 min with a solution containing 50% ethanol and 1% acetic acid to monitor absorbance at 540 nm.

Each serum sample was evaluated in duplicates using serial 2-fold dilutions. Controls included uninfected cells ("mock-infected"), and infected cells to which only vehicle was added. Full curves of positive plasma from convalescent patient (COV(+)) and GS-441524 were run as positive control inhibitors. GS-441524 is the main metabolite of remdesivir, a broad-spectrum antiviral that blocks the RNA polymerase of SARS-CoV-2.

The average absorbance at 540 nm (A540) observed in infected cells (in the presence of vehicle alone) was calculated and then subtracted from all samples to determine the inhibition of the virus induced CPE. Data points were then normalized to the average A540 signal observed in uninfected cells ("mock") after subtraction of the absorbance signal observed in infected cells. In the neutral red CPE-based assay, uninfected cells remained viable and uptake the dye at higher levels than non-viable cells. In the absence of an antiviral agent, the virus-induced CPE kills infected cells and leads to lower A540 (this value equals 0% inhibition). By contrast, incubation with an antiviral agent (COV(+) or GS-441524) prevents the virus induced CPE and leads absorbance levels similar to those observed in uninfected cells. Full recovery of cell viability in infected cells represent 100% inhibition of virus replication.

### Viability Assay:

Uninfected cells were incubated with the same eight concentrations of a serum sample as used in the antiviral assays. The incubation temperature and duration of the incubation period mirrored the conditions of the prevention of virus-induced CPE assay, and cell viability was evaluated with the same neutral red uptake method as used in the antiviral assays. The extent of viability was monitored by measuring absorbance at 540 nm. When analyzing the data, background levels obtained from wells with no cells were subtracted from all data-points. Absorbance readout values were given as a percentage of the average signal observed in uninfected cells treated with vehicle alone.

### Results:

Antiviral effect was observed for Samples 1 and 3 at 1:8 and 1:32 dilutions. The antiviral effect was stronger when the serum samples were pre-incubated with the Vero E6 cells (Protocol 1) than when the serum samples were added after viral adsorption (Protocol 2). In Protocol 1, Samples 2 and 3 brought the levels of neutral red uptake to about 25% and about 40%, respectively, as compared with uninfected cells. As expected, Samples 2 and 4 did not display significant inhibition of the virus-induced CPE in Protocol 1.

When the serum samples were assessed with Protocol 2, some prevention of the CPE was observed with Sample 4, but neutral red uptake only reached 25-30% of the level observed in uninfected cells. No effect was observed with Samples 1, 2, and 3 when the serum samples were added after viral adsorption.

No cytotoxicity was observed in the viability assay at the concentrations evaluated for each serum sample.

Sequences described herein are provided in the sequence table below.

### SEQUENCE TABLE

| **SEQ ID NO:** | **Description** | **Amino Acid Sequence** |
|---|---|---|
| 1 | Poliovirus RDRP | EGSKEPAVLNPKDPRLKTDFEEAIFSKYTGNKIML MDEYMEEAVDHYVGCLEPLDISVDPIPLESAMYG MDGLEALDLTTSAGFPYLLQGKKKRDIFNRHTRDT TEMTKMLEKYGVDLPFVTFVKDELRSREKVEKGK SRLIEASSLNDSVAMRVAFGNLYATFHSNPGTATG SAVGCDPDIFWSKIPILLDGEIFAFDYTGYDASLSPV WFACLKKVLIKLGYTHQTSFIDYLCHSVHLYKDRK YIVNGGMPSGSSGTSIFNTMINNIIIRTLLIRVYKGID LDQFKMIAYGDDVIASYPHKIDPGLLAEAGKHYGL VMTPADKGTSFVDTNWENVTFLKRYFRADDQYPF LIHPVMPMKEIHESIRWTKDPRNTQDHVRSLCYLA WHNGEEAYDEFCRKIRSVPVGRALTLPAYSSLRRK WLDSFLER |
| 2 | COVID-19 RDRP (Full Length) | SADAQSFLNRVCGVSAARLTPCGTGTSTDVVYRAF DIYNDKVAGFAKFLKTNCCRFQEKDEDDNLIDSYF VVKRHTFSNYQHEETIYNLLKDCPAVAKHDFFKFR IDGDMVPHISRQRLTKYTMADLVYALRHFDEGNC DTLKEILVTYNCCDDDYFNKKDWYDFVENPDILRV YANLGERVRQALLKTVQFCDAMRNAGIVGVLTLD NQDLNGNWYDFGDFIQTTPGSGVPVVDSYYSLLM PILTLTRALTAESHVDTDLTKPYIKWDLLKYDFTEE RLKLFDRYFKYWDQTYHPNCVNCLDDRCILHCAN FNVLFSTVFPPTSFGPLVRKIFVDGVPFVVSTGYHF RELGVVHNQDVNLHSSRLSFKELLVYAADPAMHA ASGNLLLDKRTTCFSVAALTNNVAFQTVKPGNFNK DFYDFAVSKGFFKEGSSVELKHFFFAQDGNAAISD YDYYRYNLPTMCDIRQLLFVVEVVDKYFDCYDGG CINANQVIVNNLDKSAGFPFNKWGKARLYYDSMS YEDQDALFAYTKRNVIPTITQMNLKYAISAKNRAR TVAGVSICSTMTNRQFHQKLLKSIAATRGATVVIGT SKFYGGWHNMLKTVYSDVENPHLMGWDYPKCDR AMPNMLRIMASLVLARKHTTCCSLSHRFYRLANEC AQVLSEMVMCGGSLYVKPGGTSSGDATTAYANSV FNICQAVTANVNALLSTDGNKIADKYVRNLQHRL YECLYRNRDVDTDFVNEFYAYLRKHFSMMILSDD AVVCFNSTYASQGLVASIKNFKSVLYYQNNVFMSE AKCWTETDLTKGPHEFCSQHTMLVKQGDDYVYLP |
| | | YPDPSRILGAGCFVDDIVKTDGTLMIERFVSLAIDA YPLTKHPNQEYADVFHLYLQYIRKLHDELTGHML DMYSVMLTNDNTSRYWEPEFYEAMYTPHTVLQ |
| 3 | COVID-19 RDRP-1 | SADAQSFLNRVCGVSAARLTPCGTGTSTDVVYRAF DIYNDKVAGFAKFLKTNCCRFQEKDEDDNLIDSYF VVKRHTFSNYQHEETIYNLLKDCPAVAKHDFFKFR IDGDMVPHISRQRLTKYTMADLVYALRHFDEGNC DTLKEILVTYNCCDDDYFNKKDWYDFVENPDILRV YANLGERVRQALLKTVQFCDAMRNAGIVGVLTLD NQDLNGNWYDFGDFIQTTPGSGVPVVDSYYSLLM PILTLTRALTAESHVDTDLTKPYIKWDLLKYDFTEE RLKLFDRYFKYWDQTYHPNCVNCLDDRCILHCAN FNVLFSTVFPPTSFGPLVR |
| 4 | COVID-19 RDRP-2 | KIFVDGVPFVVSTGYHFRELGVVHNQDVNLHSSRL SFKELLVYAADPAMHAASGNLLLDKRTTCFSVAA LTNNVAFQTVKPGNFNKDFYDFAVSKGFFKEGSSV ELKHFFFAQDGNAAISDYDYYRYNLPTMCDIRQLL FVVEVVDKYFDCYDGGCINANQVIVNNLDKSAGF PFNKWGKARLYYDSMSYEDQDALFAYTKRNVIPTI TQMNLKYAISAKNRARTVAGVSICSTMTNRQFHQ KLLKSIAATRGATVVIGTSKFYGGWHNMLKTVYS DVENPHLMGWDYPKCDRAMPNMLRIMASLVLAR KHTTCCSLSHRFYRLANECAQVLSEMVMCGGSLY VKPGGTSSGDATTAYANSVFNICQAVTANVNALLS TDGNKIADKYVRNLQHRLYECLYRNRDVDTDFVN EFYAYLRKHFSMMILSDDAVVCFNSTYASQGLVAS IKNFKSVLYYQNNVFMSEAKCWTETDLTKGPHEFC SQHTMLVKQGDDYVYLPYPDPSRILGAGCFVDDIV KTDGTLMIERFVSLAIDAYPLTKHPNQEYADVFHL YLQYIRKLHDELTGHMLDMYSVMLTNDNTSRYW EPEFYEAMYTPHTVLQ |

The examples set forth above are provided to give those of ordinary skill in the art with a complete disclosure and description of how to make and use the claimed embodiments, and are not intended to limit the scope of what is disclosed herein.

## Claims

1. A poliomyelitis vaccine for use in (i) preventing a person from an infection by a *Coronaviridae* virus, (ii) inducing a protective immune response in a person against a *Coronaviridae* virus, (iii) inducing an immune response in a person protective against severe acute respiratory syndrome caused by an infection of a *Coronaviridae* virus, or (iv) reducing the severity of one or more symptoms of an infectious disease caused by a *Coronaviridae* virus in a person; wherein the poliomyelitis vaccine is an inactivated poliomyelitis vaccine.

2. The poliomyelitis vaccine for use of claim 1, wherein the poliomyelitis vaccine is for use in preventing a person from an infection by a *Coronaviridae* virus.

3. The poliomyelitis vaccine for use of claim 1 or 2, wherein the *Coronaviridae* virus is a *Coronavirinae* virus.

4. The poliomyelitis vaccine for use of any one of claims 1 to 3, wherein the *Coronaviridae* virus is a severe acute respiratory syndrome coronavirus-1, a severe acute respiratory syndrome coronavirus-2, or a Middle East respiratory syndrome-related coronavirus.

5. The poliomyelitis vaccine for use of any one of claims 1 to 4, wherein the *Coronaviridae* virus is a severe acute respiratory syndrome coronavirus-2.

6. The poliomyelitis vaccine for use of any one of claims 1 to 5, wherein the inactivated poliomyelitis vaccine comprises inactivated Type 1 (Mahoney) poliovirus, inactivated Type 2 (MEF-1) poliovirus, inactivated Type 3 (Saukett) poliovirus, or a mixture thereof.

7. The poliomyelitis vaccine for use of any one of claims 1 to 6, wherein the inactivated poliomyelitis vaccine comprises inactivated Type 1 poliovirus, inactivated Type 2 poliovirus, and inactivated Type 3 poliovirus; or wherein the inactivated poliomyelitis vaccine comprises about 40 D antigen units of inactivated Type 1 poliovirus, about 8 D antigen units of inactivated Type 2 poliovirus, and 32 D antigen units of inactivated Type 3 poliovirus.

8. The poliomyelitis vaccine for use of any one of claims 1 to 7, wherein the poliomyelitis vaccine is formulated for oral or parenteral administration.

9. The poliomyelitis vaccine for use of any one of claims 1 to 8, wherein the poliomyelitis vaccine is formulated for oral administration.

10. The poliomyelitis vaccine for use of any one of claims 1 to 8, wherein the poliomyelitis vaccine is formulated for parenteral administration.

11. The poliomyelitis vaccine for use of any one of claims 1 to 8 and 10, wherein the poliomyelitis vaccine is formulated for intradermal, intramuscular, or subcutaneous administration.

## Patentansprüche

1. Poliomyelitis-Impfstoff zur Verwendung bei (i) der Prävention einer Infektion einer Person durch ein *Coronaviridae-Virus*, (ii) der Induzierung einer schützenden Immunreaktion in einer Person gegen ein *Coronaviridae-Virus*, (iii) der Induzierung einer Immunreaktion in einer Person zum Schutz gegen ein schweres akutes respiratorisches Syndrom, verursacht durch eine Infektion mit einem *Coronaviridae-Virus*, oder (iv) der Verringerung der Schwere eines oder mehrerer Symptome einer Infektionskrankheit, verursacht durch ein *Coronaviridae-Virus* in einer Person; wobei der Poliomyelitis-Impfstoff ein inaktivierter Poliomyelitis-Impfstoff ist.

2. Der Poliomyelitis-Impfstoff zur Verwendung gemäß Anspruch 1, wobei der Poliomyelitis-Impfstoff zur Verwendung bei der Prävention einer Infektion einer Person durch ein *Coronaviridae-Virus* ist.

3. Der Poliomyelitis-Impfstoff zur Verwendung gemäß Anspruch 1 oder 2, wobei das *Coronaviridae-Virus* ein *Coronavirinae-Virus* ist.

4. Der Poliomyelitis-Impfstoff zur Verwendung gemäß einem der Ansprüche 1 bis 3, wobei das *Coronaviridae-Virus* ein Coronavirus-1 des schweren akuten respiratorischen Syndroms, ein Coronavirus-2 des schweren akuten respiratorischen Syndroms oder ein "*Middle East respiratory syndrome-related*" Coronavirus ist.

5. Der Poliomyelitis-Impfstoff zur Verwendung gemäß einem der Ansprüche 1 bis 4, wobei das *Coronaviridae-Virus* ein Coronavirus-2 des schweren akuten respiratorischen Syndroms ist.

6. Der Poliomyelitis-Impfstoff zur Verwendung gemäß einem der Ansprüche 1 bis 5, wobei der inaktivierte Poliomyelitis-Impfstoff ein inaktiviertes Poliovirus Typ 1 (Mahoney), ein inaktiviertes Poliovirus Typ 2 (MEF-1), ein inaktiviertes Poliovirus Typ 3 (Saukett) oder eine Mischung davon umfasst.

7. Der Poliomyelitis-Impfstoff zur Verwendung gemäß einem der Ansprüche 1 bis 6, wobei der inaktivierte Poliomyelitis-Impfstoff inaktiviertes Poliovirus Typ 1, inaktiviertes Poliovirus Typ 2 und inaktiviertes Poliovirus Typ 3 umfasst; oder wobei der inaktivierte Poliomyelitis-Impfstoff ungefähr 40 D-Antigeneinheiten des inaktivierten Poliovirus Typ 1, ungefähr 8 D-Antigeneinheiten des inaktivierten Poliovirus Typ 2 und 32 D-Antigeneinheiten des inaktivierten Poliovirus Typ 3 umfasst.

8. Der Poliomyelitis-Impfstoff zur Verwendung gemäß einem der Ansprüche 1 bis 7, wobei der Poliomyelitis-Impfstoff zur oralen oder parenteralen Verabreichung formuliert ist.

9. Der Poliomyelitis-Impfstoff zur Verwendung gemäß einem der Ansprüche 1 bis 8, wobei der Poliomyelitis-Impfstoff zur oralen Verabreichung formuliert ist.

10. Der Poliomyelitis-Impfstoff zur Verwendung gemäß einem der Ansprüche 1 bis 8, wobei der Poliomyelitis-Impfstoff zur parenteralen Verabreichung formuliert ist.

11. Der Poliomyelitis-Impfstoff zur Verwendung gemäß einem der Ansprüche 1 bis 8 und 10, wobei der Poliomyelitis-Impfstoff zur intradermalen, intramuskulären oder subkutanen Verabreichung formuliert ist.

## Revendications

1. Vaccin contre la poliomyélite destiné à être utilisé dans (i) la prévention d'une personne contre une infection par un virus *Coronaviridae*, (ii) l'induction chez une personne d'une réponse immune protectrice contre un virus *Coronaviridae*, (iii) l'induction chez une personne d'une réponse immune protectrice contre le syndrome respiratoire aigu sévère causé par une infection d'un virus *Coronaviridae*, ou (iv) la réduction de la sévérité d'un ou de plusieurs symptômes d'une maladie infectieuse causée par un virus *Coronaviridae* chez une personne ; sachant que le vaccin contre la poliomyélite est un vaccin contre la poliomyélite inactivé.

2. Le vaccin contre la poliomyélite destiné à être utilisé selon la revendication 1, sachant que le vaccin contre la poliomyélite est destiné à être utilisé dans la prévention d'une personne contre une infection par un virus *Coronaviridae.*

3. Le vaccin contre la poliomyélite destiné à être utilisé selon la revendication 1 ou 2, sachant que le virus *Coronaviridae* est un virus *Coronavirinae.*

4. Le vaccin contre la poliomyélite destiné à être utilisé selon l'une quelconque des revendications 1 à 3, sachant que le virus *Coronaviridae* est un coronavirus-1 à syndrome respiratoire aigu sévère, un coronavirus-2 à syndrome respiratoire aigu sévère, ou un coronavirus relatif au syndrome respiratoire du Moyen-Orient.

5. Le vaccin contre la poliomyélite destiné à être utilisé selon l'une quelconque des revendications 1 à 4, sachant que le virus *Coronaviridae* est un coronavirus-2 à syndrome respiratoire aigu sévère.

6. Le vaccin contre la poliomyélite destiné à être utilisé selon l'une quelconque des revendications 1 à 5, sachant que le vaccin contre la poliomyélite inactivé comprend un poliovirus de type 1 (Mahoney) inactivé, un poliovirus de type 2 (MEF-1) inactivé, un poliovirus de type 3 (Saukett) inactivé, ou un mélange de ceux-ci.

7. Le vaccin contre la poliomyélite destiné à être utilisé selon l'une quelconque des revendications 1 à 6, sachant que le vaccin contre la poliomyélite inactivé comprend un poliovirus de type 1 inactivé, un poliovirus de type 2 inactivé, et un poliovirus de type 3 inactivé ; ou sachant que le vaccin contre la poliomyélite inactivé comprend environ 40 unités d'antigène D de poliovirus de type 1 inactivé, environ 8 unités d'antigène D de type 2 inactivé, et 32 unités d'antigène D de poliovirus de type 3 inactivé.

8. Le vaccin contre la poliomyélite destiné à être utilisé selon l'une quelconque des revendications 1 à 7, sachant que le vaccin contre la poliomyélite est formulé pour administration orale ou parentérale.

9. Le vaccin contre la poliomyélite destiné à être utilisé selon l'une quelconque des revendications 1 à 8, sachant que le vaccin contre la poliomyélite est formulé pour administration orale.

10. Le vaccin contre la poliomyélite destiné à être utilisé selon l'une quelconque des revendications 1 à 8, sachant que le vaccin contre la poliomyélite est formulé pour administration parentérale.

11. Le vaccin contre la poliomyélite destiné à être utilisé selon l'une quelconque des revendications 1 à 8 et 10, sachant que le vaccin contre la poliomyélite est formulé pour administration intradermique, intramusculaire, ou sous-cutanée.
